# EUROPEAN PATENT APPLICATION

(11) **EP 3 884 925 A1**
(43) Date of publication of application: **29.09.2021**
(21) Application number: 19886281.5
(22) Date of filing: 19.11.2019
(51) Int. Cl.: A61K 8/19, A61Q 17/04

(54) **SUNSCREEN COMPOSITION COMPRISING SURFACE-DEFECTED CERIUM OXIDE PARTICLES, AND PREPARATION METHOD THEREFOR**

(30) Priority: 19.11.2018 KR 20180142915
(71) Applicant: Soulbrain Co., Ltd., Seongnam-si, Gyeonggi-do 13486 (KR)
(72) Inventor: KIM, Seok Joo, Seongnam-si Gyeonggi-do 13486 (KR); KIM, Hae Chun, Seongnam-si Gyeonggi-do 13486 (KR); LEE, Jeong Ho, Seongnam-si Gyeonggi-do 13486 (KR)
(74) Representative: M. Zardi & Co S.A.
(86) International application number: PCT/KR2019/015886
(87) International publication number: WO 2020/106037

(57) **Abstract**

Disclosed are a sunscreen composition containing surface-defected cerium oxide particles and a method for preparing the sunscreen composition. The sunscreen composition containing surface-defected cerium oxide particles has excellent ability to kill bacteria, may have a high sun protection factor (SPF) and a high PA index, and may exhibit excellent dispersion stability since the layer separation thereof does not occur even after a long period of time elapses.

## Description

### [Technical Field]

The present invention relates to a sunscreen composition containing surface-defected cerium oxide particles and a method for preparing the sunscreen composition.

### [Background Art]

Since the first cosmetic product containing a sunscreen agent was developed in the United States in 1928, the demand for sunscreen agents has steadily increased. Sunscreen agents are intended to prevent skin cancer, sunburn, and photoaging caused by ultraviolet rays. Recently, interest in prevention of photoaging through blocking of ultraviolet rays corresponding to UVA1 and UVA2 wavelengths is increasing for cosmetic purposes. Ultraviolet blocking functions are imparted to most formulations such as BB cream, CC cream, cushion, sun spray, and sun stick in addition to sun cream.

In order to block ultraviolet rays, sunscreen agents are added, and sunscreen agents may be divided into organic sunscreen agents and inorganic sunscreen agents. As organic sunscreen agents, there are typically chemical sunscreen agents that convert light into heat. As inorganic sunscreen agents, there are typically physical sunscreen agents that reflect, scatter, and absorb light. Unlike basic skin care cosmetics, sunscreen is mainly used to attenuate ultraviolet rays on the upper part of the epidermis, that is, the outermost part of the skin. However, in the case of organic sunscreen agents such as avobenzone, the molecular size thereof is small and there is a possibility that the organic sunscreen agent penetrates the skin. Organic sunscreen agents have advantages of having little white turbidness and various absorption wavelengths but may cause skin problems or side effects such as irritation of the eyes when applied around the eyes in the case of sensitive skin. On the other hand, inorganic sunscreen agents are relatively safe and have a favorable blocking effect but are white pigments having high refractive indices, and thus may cause problems such as white turbidness. Due to the recent nature-friendly trend of cosmetic materials, in Korea, the preference for sunscreen products of 'inorganic sunscreen' formulations containing only inorganic sunscreen agents as functional ingredients is high.

Titanium dioxide (TiO₂) and zinc oxide (ZnO) are used as inorganic sunscreen agents but have various disadvantages. First, the energy band gaps of titanium dioxide and zinc oxide are 3.0 eV and 3.2 eV, respectively, and thus titanium dioxide and zinc oxide are advantageous for UVB and UVA2 absorption but cannot absorb UVA1 that is an intermediate wavelength. Second, the refractive indices of titanium dioxide and zinc oxide are as high as 2.7 and 2.2, respectively, and thus white cloudy appearance may be noticeable when the sunscreen is applied to the skin. Third, titanium dioxide and zinc oxide have a great photocatalytic effect to decompose or denature organic materials, particularly coloring matters, under light energy and thus may cause ingredient denaturation of the formulation and pigmentation. In particular, when the photocatalytic effect is great, the surfaces of titanium dioxide and zinc oxide are required to be covered with a second material for safety reasons. In the case of titanium dioxide, aluminum oxide (Al₂O₃) or silicon dioxide (SiO₂) is used to cover 20 parts by weight or more of titanium dioxide. However, when the surface of titanium dioxide is covered with aluminum oxide and silicon dioxide, there may be disadvantages that the powder texture is heavy, the sunscreen is not smoothly applied, and the feel of use is stiff. Hence, it is required to develop a sunscreen composition that can compensate for the above disadvantages.

Accordingly, the present inventors have studied to solve the above problems, found out that a sunscreen composition which can absorb UVA1, suppresses white turbidness by a low refractive index, and is stable because of a low photocatalytic effect can be formed when cerium oxide having the surface modified with a fatty acid is used in the sunscreen composition, and applied for this sunscreen composition (Korean Patent Application No. 10-2017-0142617).

After the above patent application, the present inventors have continued to carry out related studies, prepared a sunscreen composition containing surface-defected cerium oxide particles including Ce³⁺ by generating surface defects on cerium oxide particles in the process of related studies, and completed the present invention by discovering that the sunscreen composition has excellent ability to kill bacteria.

In this regard, Korean Patent Registration No. 10-0569083 discloses a metal oxide doped cerium oxide, a method for producing the same, a resin composition and a cosmetic composition containing the same.

### [Summary of Invention]

### [Technical Problem]

The present invention has been devised to solve the above-described problems, and an embodiment of the present invention provides a sunscreen composition containing surface-defected cerium oxide particles.

Another embodiment of the present invention provides a method for preparing the sunscreen composition.

The technical problem to be achieved by the present invention is not limited to the technical problems mentioned above, and other technical problems that are not mentioned will be clearly understood by those skilled in the technical field to which the present invention pertains from the following description.

### [Solution to Problem]

As a technical means for achieving the above-described technical problems, an aspect of the present invention provides a sunscreen composition containing surface-defected cerium oxide particles including Ce⁴⁺ and Ce³⁺.

A peak may be detected at 550 cm⁻¹ to 650 cm⁻¹ when the cerium oxide particles are analyzed by Raman spectroscopy.

The weight ratio of Ce³⁺ to Ce⁴⁺ may be 1 : 0.3 to 0.8.

The cerium oxide particles may have a surface modified with a saturated fatty acid having 10 to 30 carbon atoms, an unsaturated fatty acid having 10 to 30 carbon atoms, or polyhydroxystearic acid.

The saturated fatty acid having 10 to 30 carbon atoms may include a fatty acid selected from the group consisting of capric acid, undecylic acid, lauric acid, tridecylic acid, myristic acid, palmitic acid, margaric acid, stearic acid, nonadecylic acid, arachidic acid, heneicosanoic acid, behenic acid, tricosanoic acid, lignoceric acid, pentacosanoic acid, cerotic acid, heptacosanoic acid, montanic acid, nonacosanoic acid, melissic acid, and combinations thereof.

A ratio of the number of carbon atoms and the number of double bonds may be 18:1 to 18:3 in the unsaturated fatty acid having 10 to 30 carbon atoms.

The unsaturated fatty acid having 10 to 30 carbon atoms may include a fatty acid selected from the group consisting of α-linolenic acid, linoleic acid, γ-linolenic acid, dihomo-γ-linolenic acid, palmitoleic acid, vaccenic acid, oleic acid, trans-elaidic acid, and combinations thereof.

The content of the saturated fatty acid, unsaturated fatty acid, or polyhydroxystearic acid may be 1 part by weight to 10 parts by weight with respect to 100 parts by weight of the surface-modified cerium oxide (CeO₂) particles.

The content of the cerium oxide particles may be 5 parts by weight to 30 parts by weight with respect to 100 parts by weight of the entire sunscreen composition.

The primary particle size of the cerium oxide (CeO₂) may be 10 to 30 nm, the secondary particle size of the cerium oxide (CeO₂) may be 100 to 200 nm, and the ratio of the secondary particle size to the primary particle size may be 3 to 20.

The sunscreen composition may further contain an organic sunscreen agent.

The organic sunscreen agent may include a material selected from the group consisting of octyl methoxycinnamate, ethylhexylsalicylate, homosalate, 4-methylbenzylidene camphor, drometrizole, drometrizole trisiloxane, digalloyl trioleate, disodium phenyl dibenzimidazole tetrasulfonate acid, diethylamino hydroxybenzoyl hexyl benzoate, diethylhexyl butamido triazone, methylene bis-benzotriazolyl tetramethylbutylphenol, menthyl anthranilate, benzophenone-3, benzophenone-4, benzophenone-8, butyl methoxydibenzoylmethane, bis-ethylhexyloxyphenolmethoxyphenyl triazine, cinoxate, ethylhexyl dimethyl PABA, ethylhexyl methoxycinnamate, ethylhexyl triazone, octocrylene, isoamyl p-methoxycinnamate, terephthalylidene dicamphor sulfonic acid, phenylbenzimidazole sulfonic acid, polysilicone-15, and combinations thereof.

Another aspect of the present invention provides a method for preparing a sunscreen composition, which includes: performing hydrothermal synthesis of a material selected from the group consisting of cerium hydroxide, cerium oxide, cerium carbonate, cerium nitrate, cerium chloride, ammonium cerium nitrate, and combinations thereof to obtain cerium oxide (CeO₂) particles; and adding a metal oxide solution to the obtained cerium oxide (CeO₂) particles and performing hydrothermal synthesis of the mixture to prepare surface-defected cerium oxide particles including Ce³⁺.

The metal oxide in the metal oxide solution may include a material selected from the group consisting of alumina (Al₂O₃), ceria (CeO₂), silica (SiO₂), zirconia (ZrO₂), titania (TiO₂), germania (GeO₂), and combinations thereof.

The method for preparing a sunscreen composition may further include: adding the prepared surface-defected cerium oxide particles to an aqueous medium; and milling the aqueous medium after the step of preparing surface-defected cerium oxide particles.

The method for preparing a sunscreen composition may further include: stirring a saturated fatty acid or an unsaturated fatty acid into the prepared surface-defected cerium oxide particles to obtain cerium oxide (CeO₂) particles having surfaces modified with a saturated fatty acid having 10 to 30 carbon atoms or an unsaturated fatty acid having 10 to 30 carbon atoms or stirring substituted or unsubstituted polyhydroxystearic acid into the prepared surface-defected cerium oxide particles to obtain cerium oxide (CeO₂) particles having surfaces modified with polyhydroxystearic acid; dispersing the cerium oxide (CeO₂) particles having surfaces modified with a fatty acid or polyhydroxystearic acid in an organic solvent; and milling the organic solvent after the step of preparing surface-defected cerium oxide particles.

### [Advantageous Effects of Invention]

According to an embodiment of the present invention, the sunscreen composition containing surface-defected cerium oxide particles has excellent ability to kill bacteria, may have a high sun protection factor (SPF) and a high PA index, and may exhibit excellent dispersion stability since the layer separation thereof does not occur even after a long period of time elapses.

The sunscreen composition has a high dynamic viscosity in the low frequency region and the high frequency region and thus the formulation thereof exhibits excellent emulsification/dispersion phase-stability, and the sunscreen composition exhibits excellent application property and thus can be usefully used as a cosmetic composition for ultraviolet blocking.

The sunscreen composition according to an embodiment of the present invention is capable of absorbing UVA1 of the intermediate ultraviolet wavelength region and thus can absorb ultraviolet rays in the entire UVA1 to UVA2 region, and the sunscreen composition has a high sun protection factor and thus has an excellent ultraviolet blocking effect.

The sunscreen composition according to an embodiment of the present invention does not cause white cloudy appearance when applied to the skin since the particles have a low light refractive index, and thus can be used as a cosmetic composition for ultraviolet blocking that provides natural impression of color.

The sunscreen composition according to an embodiment of the present invention contains cerium oxide particles having the surfaces modified with a fatty acid or polyhydroxystearic acid, thus exhibits high emulsifying property in oil-in-water, water-in-oil, and non-aqueous formulations, and can be used to prepare various formulations of cosmetic compositions for ultraviolet blocking.

The effects of the present invention are not limited to the above effects, and should be understood to include all effects that can be deduced from the configuration of the invention described in the detailed description or claims of the present invention.

### [Brief Description of Drawings]

FIG. 1 a schematic diagram illustrating surface-defected cerium oxide particles according to an embodiment of the present invention;
FIG. 2 is a graph illustrating analysis results of surface-defected cerium oxide particles according to Example of the present invention by Raman spectroscopy;
FIG. 3A is a graph illustrating analysis results of surface-defected cerium oxide particles according to Comparative Example of the present invention by Raman spectroscopy;
FIG. 3B is a graph illustrating analysis results of cerium oxide particles including Ce³⁺ and Ce⁴⁺ according to Example of the present invention by Raman spectroscopy; and
FIG. 4 is a photograph illustrating the ability of the sunscreen composition according to Example of the present invention to kill bacteria.

### [Description of Embodiments]

Hereinafter, the present invention will be described in more detail. However, the present invention can be implemented in several different forms. The present invention is not limited by the embodiments described herein, and the present invention is only defined by the claims to be described later.

In addition, terms used in the present invention are used only to describe specific embodiments, and are not intended to limit the present invention. Singular expressions include plural expressions unless the context clearly indicates otherwise. In the entire specification of the present invention, "including" a certain component means that other components may be further included rather than excluding other components unless specifically stated to the contrary.

A first aspect of the present application provides a sunscreen composition containing surface-defected cerium oxide particles including Ce⁴⁺ and Ce³⁺.

Hereinafter, the sunscreen composition according to the first aspect of the present application will be described in detail.

In an embodiment of the present application, Ce⁴⁺ may mean existing cerium oxide particles and Ce³⁺ may mean surface-defected cerium oxide particles. At this time, the surface defect may be achieved by treating cerium oxide particles with a metal oxide.

In an embodiment of the present application, the metal oxide in the metal oxide solution may include a material selected from the group consisting of alumina (Al₂O₃), ceria (CeO₂), silica (SiO₂), zirconia (ZrO₂), titania (TiO₂), germania (GeO₂), and combinations thereof. The content of the metal oxide may be 0.001 part by weight to 1 part by weight, preferably 0.01 part by weight with respect to 100 parts by weight of the cerium oxide particles.

In an embodiment of the present application, a peak may be detected at 550 cm⁻¹ to 650 cm⁻¹, preferably at 595 cm⁻¹ when the cerium oxide particles are analyzed by Raman spectroscopy.

In an embodiment of the present application, the weight ratio of Ce³⁺ to Ce⁴⁺ may be 1:0.3 to 0.8.

In an embodiment of the present application, the cerium oxide particles may be prepared from a cerium precursor such as a material selected from the group consisting of cerium hydroxide, cerium oxide, cerium carbonate, cerium nitrate, cerium chloride, ammonium cerium nitrate, and combinations thereof. All cerium oxide particles prepared by ordinary cerium oxide preparation methods may be used without particular limitation.

In an embodiment of the present application, the cerium oxide particles may be a cubic, hexagonal, polygonal, spherical, or aggregated spherical shape and may be a mixture of cerium oxide particles having the shapes, but the form and shape of the cerium oxide particles are not limited to the kinds.

In an embodiment of the present application, the content of the cerium oxide particles may be 5 parts by weight to 30 parts by weight, preferably 10 parts by weight to 20 parts by weight, more preferably 20 parts by weight with respect to 100 parts by weight of the entire sunscreen composition. When the content of the cerium oxide particles is less than 5 parts by weight, the content of the cerium oxide particles is too low, the wavelengths in the UVA1 region may not be absorbed, and thus the effects of the sunscreen composition according to the present invention may not be exerted. When the content of the cerium oxide particles exceeds 30 parts by weight, the solid content is too high such that the viscosity of the cosmetic may become too high, and thus the application property may be impaired. In addition, when the content of the cerium oxide particles is less than 5 parts by weight, it may be difficult to expect the ultraviolet blocking effect.

In an embodiment of the present application, the purity of the powder of the cerium oxide particles may be 90% to 99.99%, preferably 95% to 99.9%, more preferably 98% to 99.9%. When the purity of the powder of the cerium oxide particles is less than 98%, the skin stability of the sunscreen composition may be deteriorated by by-products other than the cerium oxide particles.

In an embodiment of the present application, the zeta potential value of surface charge of the cerium oxide particles may be 10 to 60 mV, preferably 20 to 50 mV, more preferably 30 to 50 mV. When the zeta potential value of surface charge of the cerium oxide particles is less than 10 mV, dispersibility may be weakened by ion repulsion. When the zeta potential value of surface charge of the cerium oxide particles exceeds 60 mV, the cerium oxide particles may be reaggregated because of excessively high charge.

In an embodiment of the present application, the primary particle size of the cerium oxide (CeO₂) may be 3 to 35 nm, 5 to 32 nm, or preferably 10 to 30 nm. The secondary particle size of the cerium oxide (CeO₂) may be 100 to 200 nm, preferably 100 to 150 nm. The ratio of the secondary particle size to the primary particle size may be 1 to 55, 3 to 50, or preferably 3 to 20. In a case where the primary particle size of the cerium oxide particles is the same, the ultraviolet blocking effect by the sunscreen composition may decrease when the secondary particle size exceeds 200 nm. When the secondary particle size of the cerium oxide particles is less than 100 nm, the cerium oxide particles are generally determined as nanoparticles and problems such as skin penetration may be caused.

In an embodiment of the present application, as the secondary particle size of the cerium oxide particles is 100 to 200 nm, the sunscreen composition containing the cerium oxide particles may have a high sun protection factor (SPF) of 20 or more and a high PA index of 10 or more, and thus may have significantly excellent efficacy.

In an embodiment of the present application, as the cerium oxide particles are contained in the sunscreen composition according to the present invention, the cerium oxide particles can absorb the wavelengths in the UVA1 region that is the intermediate ultraviolet wavelength region and thus can play a role of widening the ultraviolet blocking and absorption region of the sunscreen composition.

In an embodiment of the present application, the cerium oxide particles may be water-dispersed or oil-dispersed, and preferably water-dispersed.

In an embodiment of the present application, the water-dispersed cerium oxide particles may be dispersed in an aqueous medium. The aqueous medium may be purified water or acidic water having a pH of 5 to 7.

In an embodiment of the present application, the content of the aqueous medium may be 1 part by weight to 60 parts by weight, preferably 5 parts by weight to 55 parts by weight, more preferably 10 parts by weight to 50 parts by weight with respect to 100 parts by weight of the sunscreen composition. The content of the aqueous medium may be freely selected within the above-described range depending on the formulation of the sunscreen composition to be prepared.

In an embodiment of the present application, the aqueous medium may further contain a lower alcohol, a polyhydric alcohol, a moisturizing agent, a pH adjusting agent and the like in addition to the water-dispersed cerium oxide particles.

In an embodiment of the present application, examples of the lower alcohol include ethanol, propanol, isopropanol, isobutyl alcohol, and t-butyl alcohol. Examples of the polyhydric alcohol include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butylene-1,4-diol, hexylene glycol, and octylene glycol); and trihydric alcohols (for example, glycerin and trimethylolpropane).

In an embodiment of the present application, examples of the moisturizing agent include polyethylene glycol, propylene glycol, glycerin, 1,3-butylene glycol, xylitol, sorbitol, maltitol, chondroitin sulfuric acid, hyaluronic acid, mucoitin sulfuric acid, caronic acid, atelocollagen, cholesteryl 12-hydroxystearate, sodium lactate, bile salt, dl-pyrrolidone carboxylate, short chain soluble collagen, diglycerin (EO)PO adduct, Rosa roxburghii extract, yarrow extract, and meriroto extract. As the pH adjusting agent, for example, buffers such as lactic acid-sodium lactate, citric acid-sodium citrate, and succinic acid-sodium succinate, organic amines such as monoethanolamine, diethanolamine, triethanolamine, and tromethamine, and the like may be used.

In an embodiment of the present application, the total content of the ingredients such as the lower alcohol, polyhydric alcohol, moisturizing agent, and pH adjusting agent is not particularly limited as long as the effects of the present invention are not impaired, and may be 5 parts by weight to 25 parts by weight, preferably 10 parts by weight to 20 parts by weight, more preferably 12 parts by weight to 18 parts by weight with respect to 100 parts by weight of the sunscreen composition.

In an embodiment of the present application, the oil-dispersed cerium oxide particles may have surfaces modified with a saturated fatty acid having 10 to 30 carbon atoms, an unsaturated fatty acid having 10 to 30 carbon atoms, or polyhydroxystearic acid.

In an embodiment of the present application, the saturated fatty acid having 10 to 30 carbon atoms may include a fatty acid selected from the group consisting of capric acid, undecylic acid, lauric acid, tridecylic acid, myristic acid, palmitic acid, margaric acid, stearic acid, nonadecylic acid, arachidic acid, heneicosanoic acid, behenic acid, tricosanoic acid, lignoceric acid, pentacosanoic acid, cerotic acid, heptacosanoic acid, montanic acid, nonacosanoic acid, melissic acid, and combinations thereof.

In an embodiment of the present application, a ratio of the number of carbon atoms and the number of double bonds may be 18:1 to 18:3 in the unsaturated fatty acid having 10 to 30 carbon atoms.

In an embodiment of the present application, the unsaturated fatty acid having 10 to 30 carbon atoms may include a fatty acid selected from the group consisting of α-linolenic acid, linoleic acid, γ-linolenic acid, dihomo-γ-linolenic acid, palmitoleic acid, vaccenic acid, oleic acid, trans-elaidic acid, and combinations thereof.

In an embodiment of the present application, the saturated fatty acid having 10 to 30 carbon atoms or the unsaturated fatty acid having 10 to 30 carbon atoms is not limited to the kinds presented above, and all saturated or unsaturated fatty acids having a hydrocarbon chain having 10 to 30 carbon atoms may be included.

In an embodiment of the present application, the polyhydroxystearic acid may have a hydroxystearic acid repeating unit. At this time, the hydroxystearic acid may have a structure represented by the following Chemical Formula (1).

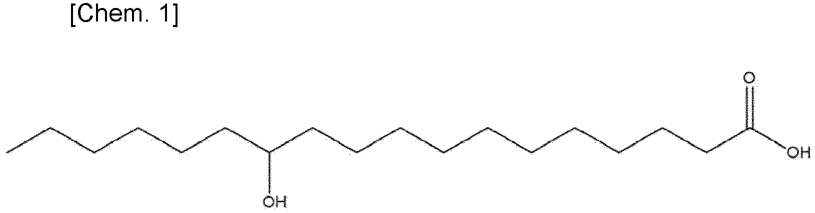

In an embodiment of the present application, the hydroxystearic acid repeating unit may have a structure represented by the following Chemical Formula (2).

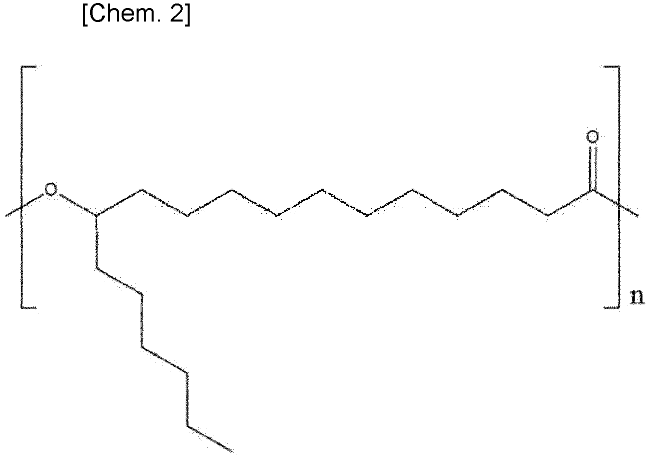

In other words, the polyhydroxystearic acid may be formed by dehydration polymerization reaction of a hydroxyl group and a carboxyl group between hydroxystearic acid monomers in a solvent such as xylene. At this time, the dehydration polymerization reaction may be performed at a high temperature of about 200°C.

In an embodiment of the present application, by modifying the surfaces of the cerium oxide particles with a fatty acid having a specific length corresponding to 10 to 30 carbon atoms or polyhydroxystearic acid, the sunscreen composition may exhibit excellent emulsification/dispersion phase-stability in an oily medium and excellent application property. When surface modification is performed with a fatty acid having 10 to 30 carbon atoms or polyhydroxystearic acid, the emulsification in an oily medium smoothly proceeds and the formulation may exhibit excellent dispersion stability.

In an embodiment of the present application, the content of the saturated fatty acid, unsaturated fatty acid, or polyhydroxystearic acid may be 0.3 part by weight to 15 parts by weight, 0.5 part by weight to 11 parts by weight, or preferably 1 part by weight to 10 parts by weight with respect to 100 parts by weight of the surface-modified cerium oxide (CeO₂) particles. When the surface is modified with less than 0.3 part by weight of the saturated fatty acid, unsaturated fatty acid, or polyhydroxystearic acid, the whole surfaces of the cerium oxide particles are not modified, and thus problems of emulsification/dispersion may occur at the time of cosmetic preparation using a solvent. When the surface is modified with more than 15 parts by weight of the saturated fatty acid, unsaturated fatty acid, or polyhydroxystearic acid, the excess fatty acid molecules that have not been used to cover the particle surfaces gather together and may act as impurities at the time of cosmetic preparation. When the content of fatty acid is too low, the particle surfaces may not be sufficiently covered. When the content of fatty acid is too high, the fatty acid molecules gather as excess molecules and act as impurities at the time of cosmetic preparation. This may lead to rancidity of the cosmetic, and excessive oiliness may diminish the feel of use.

In an embodiment of the present application, by modifying the surfaces of the cerium oxide particles with a fatty acid, the cerium oxide particles that are hydrophobic particles may exhibit excellent emulsification/dispersion phase-stability in an organic solvent.

In an embodiment of the present application, the sunscreen composition may further contain an organic sunscreen agent. In other words, by further containing an organic sunscreen agent, the sunscreen composition may have a high sun protection factor (SPF) and a high PA index.

In an embodiment of the present application, the organic sunscreen agent may include a material selected from the group consisting of octyl methoxycinnamate, ethylhexylsalicylate, homosalate, 4-methylbenzylidene camphor, drometrizole, drometrizole trisiloxane, digalloyl trioleate, disodium phenyl dibenzimidazole tetrasulfonate acid, diethylamino hydroxybenzoyl hexyl benzoate, diethylhexyl butamido triazone, methylene bis-benzotriazolyl tetramethylbutylphenol, menthyl anthranilate, benzophenone-3, benzophenone-4, benzophenone-8, butyl methoxydibenzoylmethane, bis-ethylhexyloxyphenolmethoxyphenyl triazine, cinoxate, ethylhexyl dimethyl PABA, ethylhexyl methoxycinnamate, ethylhexyl triazone, octocrylene, isoamyl p-methoxycinnamate, terephthalylidene dicamphor sulfonic acid, phenylbenzimidazole sulfonic acid, polysilicone-15, and combinations thereof.

In an embodiment of the present application, as the organic sunscreen agent, any organic compound that is harmless to the human body may be used in addition to the kinds.

In an embodiment of the present application, the content of the organic sunscreen agent may be 0 part by weight to 30 parts by weight, preferably 0 part by weight to 28 parts by weight, or more preferably 0 part by weight to 25 parts by weight with respect to 100 parts by weight of the sunscreen composition. The content of the organic sunscreen agent may be adjusted within the appropriate blending range depending on the blending limit for organic sunscreen agents in cosmetics determined by country.

In an embodiment of the present application, the sunscreen composition may further contain a cosmetic formulation composition.

In an embodiment of the present application, as the cosmetic formulation composition, ingredients usually blended in cosmetic compositions, such as fats and oils, waxes, surfactants, thickeners, coloring matters, cosmetic additives, powders, saccharides, antioxidants, buffers, various extracts, stabilizers, irritation relievers, preservatives, moisturizing agents, and fragrances, may be appropriately blended and used as long as the effects of the present invention are not impaired.

A second aspect of the present application provides a method for preparing a sunscreen composition, which includes: performing hydrothermal synthesis of a material selected from the group consisting of cerium hydroxide, cerium oxide, cerium carbonate, cerium nitrate, cerium chloride, ammonium cerium nitrate, and combinations thereof to obtain cerium oxide (CeO₂) particles; and adding a metal oxide solution to the obtained cerium oxide (CeO₂) particles and performing hydrothermal synthesis of the mixture to prepare surface-defected cerium oxide particles including Ce³⁺.

Detailed description of the parts overlapping with the first aspect of the present application has been omitted, but the contents described for the first aspect of the present application may be equally applied even if the description thereof is omitted in the second aspect.

Hereinafter, the method for preparing a sunscreen composition according to the second aspect of the present application will be described in detail.

First, in an embodiment of the present application, the method for preparing a sunscreen composition includes performing hydrothermal synthesis of a material selected from the group consisting of cerium hydroxide, cerium oxide, cerium carbonate, cerium nitrate, cerium chloride, ammonium cerium nitrate, and combinations thereof to obtain cerium oxide (CeO₂) particles.

In an embodiment of the present application, the hydrothermal synthesis is a known method, there is no particular limitation on the method, and, for example, the hydrothermal synthesis may be performed in a bottom-up manner.

In an embodiment of the present application, the cerium oxide particles may be water-dispersed or oil-dispersed, and preferably water-dispersed. However, when the cerium oxide particles are oil-dispersed, the cerium oxide particles are added to purified water and then stirred to prepare a supernatant. This is a process for dispersing cerium oxide particles in purified water, and is a process for preparing cerium oxide into a solvent phase so that the surface of cerium oxide can be evenly modified with a fatty acid or polyhydroxystearic acid.

In an embodiment of the present application, the size of the cerium oxide particles used may be 0.01 to 1 µm, 0.05 to 0.5 µm, or preferably 0.08 to 0.2 µm. The amount of purified water is preferably 2 to 10 times the weight of cerium oxide.

In an embodiment of the present application, a calcium salt may be additionally added other than the cerium precursor such as cerium hydroxide. At this time, the amount of the calcium salt added may be 0.1 part by weight to 99.9 parts by weight with respect to 100 parts by weight of the cerium precursor. At this time, the calcium salt may include, for example, a material selected from the group consisting of calcium hydroxide, calcium oxide, calcium carbonate, calcium nitrate, calcium chloride, ammonium calcium nitrate, and combinations thereof. In this case, the obtained particles may be calcium-cerium oxide particles.

In an embodiment of the present application, the obtained cerium oxide particles or calcium-cerium oxide particles may be mixed with zirconia beads at a volume ratio of 1:1. At this time, the mixing may be performed through milling using a bead mill.

Next, in an embodiment of the present application, the method for preparing a sunscreen composition includes adding a metal oxide solution to the obtained cerium oxide (CeO₂) particles and performing hydrothermal synthesis of the mixture to prepare surface-defected cerium oxide particles including Ce³⁺.

In an embodiment of the present application, cerium oxide particles including surface-defected Ce³⁺ may be prepared by treating the cerium oxide particles with a metal oxide. Hence, the prepared cerium oxide particles may include both existing Ce⁴⁺ cerium oxide particles and surface-defected Ce³⁺ cerium oxide particles.

In an embodiment of the present application, the metal oxide in the metal oxide solution may include a material selected from the group consisting of alumina (Al₂O₃), ceria (CeO₂), silica (SiO₂), zirconia (ZrO₂), titania (TiO₂), germania (GeO₂), and combinations thereof. At this time, the content of the metal oxide may be 0.001 part by weight to 1 part by weight, preferably 0.01 part by weight with respect to 100 parts by weight of the cerium oxide particles.

Next, in an embodiment of the present application, the method for preparing a sunscreen composition may further include: adding the prepared surface-defected cerium oxide particles to an aqueous medium; and milling the aqueous medium after the step of preparing surface-defected cerium oxide particles.

In an embodiment of the present application, the surface-defected cerium oxide particles may be dispersed in an aqueous medium. The aqueous medium may be purified water or acidic water having a pH of 5 to 7.

In an embodiment of the present application, the content of the aqueous medium may be 1 part by weight to 60 parts by weight, preferably 5 parts by weight to 55 parts by weight, more preferably 10 parts by weight to 50 parts by weight with respect to 100 parts by weight of the sunscreen composition. The content of the aqueous medium may be freely selected within the above-described range depending on the formulation of the sunscreen composition to be prepared.

Meanwhile, in an embodiment of the present application, the method for preparing a sunscreen composition may further include: stirring a saturated fatty acid or an unsaturated fatty acid into the prepared surface-defected cerium oxide particles to obtain cerium oxide (CeO₂) particles having surfaces modified with a saturated fatty acid having 10 to 30 carbon atoms or an unsaturated fatty acid having 10 to 30 carbon atoms or stirring substituted or unsubstituted polyhydroxystearic acid into the prepared surface-defected cerium oxide particles to obtain cerium oxide (CeO₂) particles having surfaces modified with polyhydroxystearic acid; dispersing the cerium oxide (CeO₂) particles having surfaces modified with a fatty acid or polyhydroxystearic acid in an organic solvent; and milling the organic solvent after the step of preparing surface-defected cerium oxide particles. This is to disperse the surface-defected cerium oxide particles in oil, and may be performed in response to the dispersion in water.

In an embodiment of the present application, the fatty acid or polyhydroxystearic acid may be dissolved in a solvent. At this time, the solvent is not particularly limited as long as it is an organic solvent, and for example, may be a solvent selected from the group consisting of alkyl benzoate, methylene chloride, xylene, dimethylformamide (DMF), nitrobenzene, methylethylketone, and combinations thereof. The content of the fatty acid or polyhydroxystearic acid with respect to 100 parts by weight of the solvent may be 1 part by weight to 5 parts by weight, preferably 2 parts by weight.

In an embodiment of the present application, the mixing ratio of the cerium oxide particles to the fatty acid or polyhydroxystearic acid may be 1:0.5 to 1.5, preferably 1:1.

In an embodiment of the present application, the milling is not particularly limited as long as a general milling method is used, and may be performed using, for example, a bead mill. The milling may be performed until the secondary particles of cerium oxide have a size of 100 nm to 200 nm.

Next, in an embodiment of the present application, the method for preparing a sunscreen composition may further include: mixing the aqueous medium or organic solvent with a material selected from the group consisting of silicone oil, a fiber, an emulsifier, a moisturizing agent, a plasticizer, purified water, and combinations thereof after the step of milling the aqueous medium or milling the organic solvent.

### [Examples]

Hereinafter, Examples of the present invention will be described in detail so that those skilled in the technical field to which the present invention pertains can easily implement the present invention. However, the present invention may be implemented in various different forms and is not limited to Examples described herein.

### Example Preparation of cerium oxide dispersion containing surface-defected cerium oxide particle

### Step 1: Preparation of surface-defected cerium oxide particle

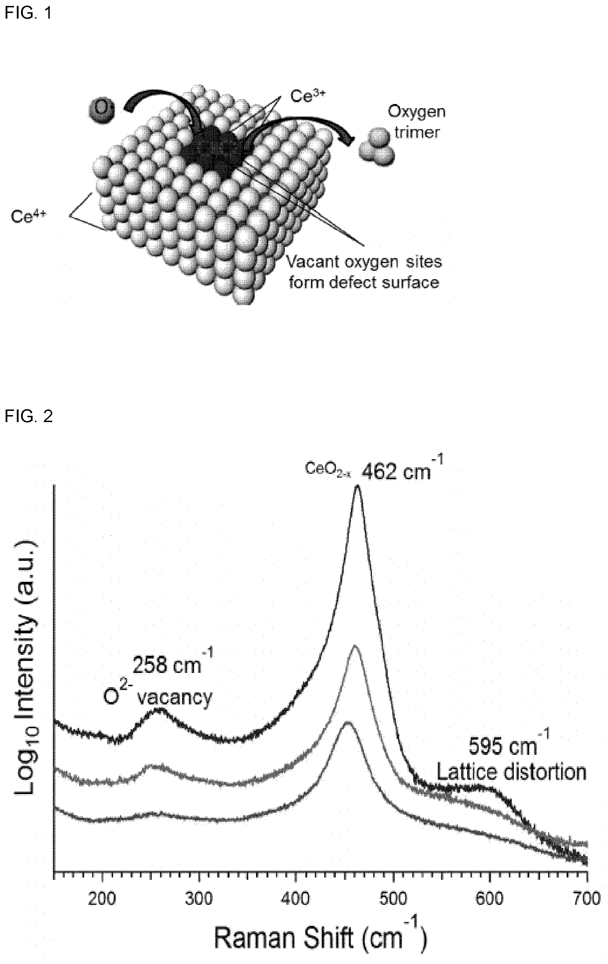

Particles were grown by hydrothermal reaction that was a chemical synthesis method using cerium nitrate as a cerium oxide precursor in a bottom-up manner. To 500 parts by weight of deionized water, 100 parts by weight of the cerium nitrate and 1 part by weight of aluminum nitrate based on 100 parts by weight of cerium nitrate were added, and 200 parts by weight of aqueous ammonia was added to the mixture while performing stirring to prepare a precursor solution having a pH of 10. The precursor solution was put into a reactor for hydrothermal synthesis and reacted at 180°C for 24 hours to prepare cerium oxide particles having surfaces defected by alumina. The particles were centrifuged to remove unreacted materials. The alumina-containing cerium oxide from which the unreacted materials had been removed contained 0.01% by weight of alumina based on 100% by weight of the entire cerium oxide particles and the primary particle size thereof was 20.1 nm. The surface-defected cerium oxide from which the unreacted materials had been removed was dried at 120°C for 4 hours to obtain a powder. A schematic diagram of the surface-defected cerium oxide particles is illustrated in FIG. 1, and a graph of analysis results of the surface-defected cerium oxide particles by Raman spectroscopy is illustrated in FIG. 2.

### Step 2: Preparation of dispersion of water-dispersed cerium oxide

To 2,500 g of deionized water, 50 g of a pH adjusting agent (nitric acid or the like) was added, and then the mixture was stirred. To the prepared solution, 2,000 g of the surface-defected cerium oxide particles prepared in step 1 was added, and then milling was performed using a bead mill to obtain a dispersion of water-dispersed cerium oxide.

### Comparative Example Preparation of dispersion of cerium oxide without surface defect

A cerium oxide dispersion was prepared in the same manner as in Example 1 except that cerium oxide was simply calcined without being treated with aluminum nitrate in the process of step 1 in Example 1.

### Experimental Example 1 Analysis by Raman spectroscopy for confirmation of surface-defected cerium oxide particles

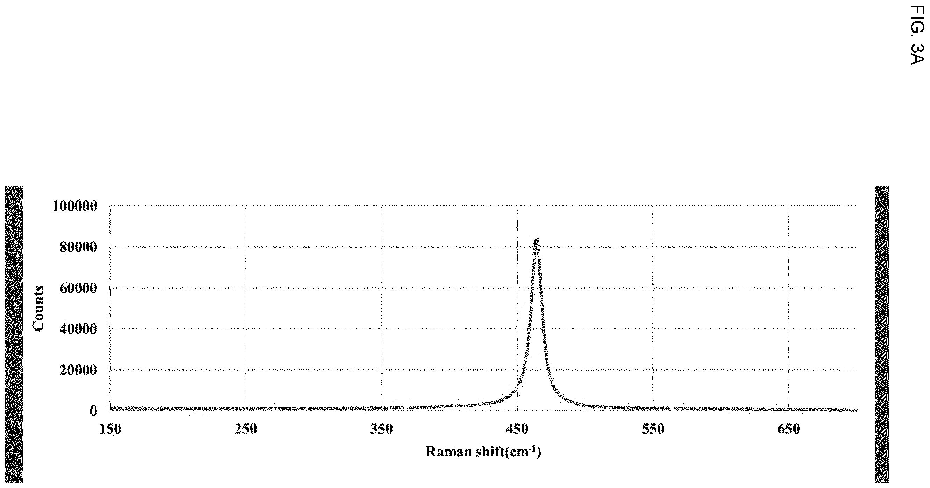

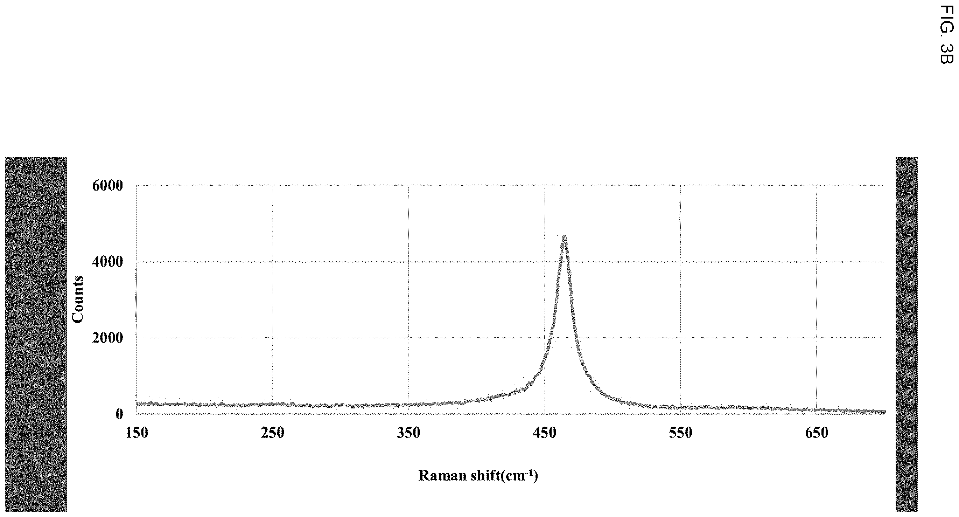

The cerium oxide particles contained in the cerium oxide dispersions of Comparative Example and Example were analyzed by Raman spectroscopy and the results are illustrated in FIGS. 3A and 3B, respectively. It has been confirmed that a peak is detected at about 460 cm⁻¹ as illustrated in FIG. 3A in the case of cerium oxide particles without surface defects but a peak is detected at about 595 cm⁻¹ as illustrated in FIG. 3B in the case of cerium oxide particles having surface defects.

### Experimental Example 2 Measurement of efficacy of cerium oxide dispersion to kill bacteria

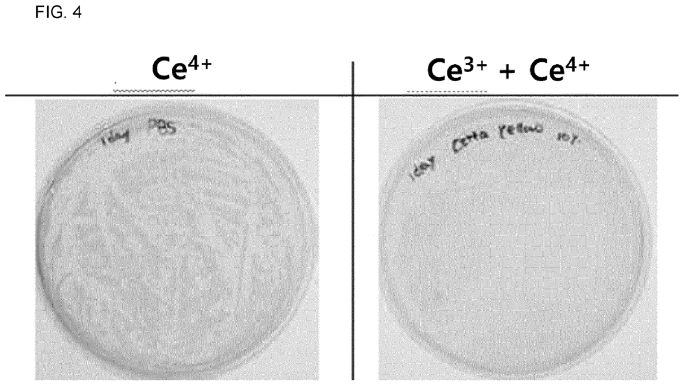

The efficacy of the cerium oxide dispersions of Comparative Example and Example to kill bacteria was measured and illustrated as a photograph in FIG. 4. As illustrated in FIG. 4, it has been confirmed that the cerium oxide dispersion corresponding to Example has superior efficacy to kill bacteria as compared to the cerium oxide dispersion corresponding to Comparative Example.

## Claims

1. A sunscreen composition comprising surface-defected cerium oxide particles including Ce⁴⁺ and Ce³⁺.

2. The sunscreen composition according to claim 1, wherein a peak is detected at 550 cm⁻¹ to 650 cm⁻¹ when the cerium oxide particles are analyzed by Raman spectroscopy.

3. The sunscreen composition according to claim 1, wherein a weight ratio of Ce³⁺ to Ce⁴⁺ is 1 : 0.3 to 0.8.

4. The sunscreen composition according to claim 1, wherein the cerium oxide particles have a surface modified with a saturated fatty acid having 10 to 30 carbon atoms, an unsaturated fatty acid having 10 to 30 carbon atoms, or polyhydroxystearic acid.

5. The sunscreen composition according to claim 4, wherein the saturated fatty acid having 10 to 30 carbon atoms includes a fatty acid selected from the group consisting of capric acid, undecylic acid, lauric acid, tridecylic acid, myristic acid, palmitic acid, margaric acid, stearic acid, nonadecylic acid, arachidic acid, heneicosanoic acid, behenic acid, tricosanoic acid, lignoceric acid, pentacosanoic acid, cerotic acid, heptacosanoic acid, montanic acid, nonacosanoic acid, melissic acid, and combinations thereof.

6. The sunscreen composition according to claim 4, wherein a ratio of the number of carbon atoms and the number of double bonds is 18:1 to 18:3 in the unsaturated fatty acid having 10 to 30 carbon atoms.

7. The sunscreen composition according to claim 4, wherein the unsaturated fatty acid having 10 to 30 carbon atoms includes a fatty acid selected from the group consisting of α-linolenic acid, linoleic acid, γ-linolenic acid, dihomo-γ-linolenic acid, palmitoleic acid, vaccenic acid, oleic acid, trans-elaidic acid, and combinations thereof.

8. The sunscreen composition according to claim 4, wherein a content of the saturated fatty acid, unsaturated fatty acid, or polyhydroxystearic acid is 1 part by weight to 10 parts by weight with respect to 100 parts by weight of the surface-modified cerium oxide (CeO₂) particles.

9. The sunscreen composition according to claim 1, wherein a content of the cerium oxide particles is 5 parts by weight to 30 parts by weight with respect to 100 parts by weight of the entire sunscreen composition.

10. The sunscreen composition according to claim 1, wherein a primary particle size of the cerium oxide (CeO₂) is 10 to 30 nm,
a secondary particle size of the cerium oxide (CeO₂) is 100 to 200 nm, and
a ratio of the secondary particle size to the primary particle size is 3 to 20.

11. The sunscreen composition according to claim 1, which further comprises an organic sunscreen agent.

12. The sunscreen composition according to claim 11, wherein the organic sunscreen agent includes a material selected from the group consisting of octyl methoxycinnamate, ethylhexylsalicylate, homosalate, 4-methylbenzylidene camphor, drometrizole, drometrizole trisiloxane, digalloyl trioleate, disodium phenyl dibenzimidazole tetrasulfonate acid, diethylamino hydroxybenzoyl hexyl benzoate, diethylhexyl butamido triazone, methylene bis-benzotriazolyl tetramethylbutylphenol, menthyl anthranilate, benzophenone-3, benzophenone-4, benzophenone-8, butyl methoxydibenzoylmethane, bis-ethylhexyloxyphenolmethoxyphenyl triazine, cinoxate, ethylhexyl dimethyl PABA, ethylhexyl methoxycinnamate, ethylhexyl triazone, octocrylene, isoamyl p-methoxycinnamate, terephthalylidene dicamphor sulfonic acid, phenylbenzimidazole sulfonic acid, polysilicone-15, and combinations thereof.

13. A method for preparing a sunscreen composition, which comprises:
performing hydrothermal synthesis of a material selected from the group consisting of cerium hydroxide, cerium oxide, cerium carbonate, cerium nitrate, cerium chloride, ammonium cerium nitrate, and combinations thereof to obtain cerium oxide (CeO₂) particles; and
adding a metal oxide solution to the obtained cerium oxide (CeO₂) particles and performing hydrothermal synthesis of the mixture to prepare surface-defected cerium oxide particles including Ce³⁺.

14. The method for preparing a sunscreen composition according to claim 13, wherein a metal oxide in the metal oxide solution includes a material selected from the group consisting of alumina (Al₂O₃), ceria (CeO₂), silica (SiO₂), zirconia (ZrO₂), titania (TiO₂), germania (GeO₂), and combinations thereof.

15. The method for preparing a sunscreen composition according to claim 13, which further comprises:
adding the prepared surface-defected cerium oxide particles to an aqueous medium; and
milling the aqueous medium
after the step of preparing surface-defected cerium oxide particles.

16. The method for preparing a sunscreen composition according to claim 13, which further comprises:
stirring a saturated fatty acid or an unsaturated fatty acid into the prepared surface-defected cerium oxide particles to obtain cerium oxide (CeO₂) particles having surfaces modified with a saturated fatty acid having 10 to 30 carbon atoms or an unsaturated fatty acid having 10 to 30 carbon atoms or stirring substituted or unsubstituted polyhydroxystearic acid into the prepared surface-defected cerium oxide particles to obtain cerium oxide (CeO₂) particles having surfaces modified with polyhydroxystearic acid;
dispersing the cerium oxide (CeO₂) particles having surfaces modified with a fatty acid or polyhydroxystearic acid in an organic solvent; and
milling the organic solvent
after the step of preparing surface-defected cerium oxide particles.
